# EUROPEAN PATENT SPECIFICATION

(11) **EP 2 524 675 B1**
(45) Date of publication and mention of the grant of the patent: **31.05.2017**
(21) Application number: 10843073.7
(22) Date of filing: 25.06.2010
(51) Int. Cl.: A61F 5/01, C22F 1/00, C22F 1/08

(54) **DEVICE FOR CORRECTING HALLUX VALGUS AND METHOD FOR PRODUCING DEVICE FOR CORRECTING HALLUX VALGUS**
VORRICHTUNG ZUR KORREKTUR VON HALLUX VALGUS UND VERFAHREN ZUR HERSTELLUNG EINER KORREKTURVORRICHTUNG FÜR HALLUX VALGUS
DISPOSITIF DE CORRECTION DE VALGUS DU GROS ORTEIL ET PROCÉDÉ DE FABRICATION DU DISPOSITIF DE CORRECTION DE VALGUS DU GROS ORTEIL

(30) Priority: 15.01.2010 JP 2010006489
(43) Date of publication of application: 21.11.2012
(73) Proprietor: Furukawa Techno Material Co., Ltd., Kanagawa 254-0016 (JP)
(72) Inventor: ISHIDA, Kiyohito, Sendai-shi Miyagi 980-0011 (JP); KAINUMA, Ryosuke, Natori-shi Miyagi 981-1231 (JP); SUTOU, Yuji, Sendai-shi Miyagi 981-0943 (JP); OMORI, Toshihiro, Sendai-shi Miyagi 981-0967 (JP); HATORI, Masahito, Sendai-shi Miyagi 989-3204 (JP)
(74) Representative: Fenlon, Christine Lesley
(86) International application number: PCT/JP2010/060820
(87) International publication number: WO 2011/086716

(56) References cited:
- WO-A1-2009/144967
- WO-A1-2009/144967
- DE-A1- 10 154 185
- DE-C- 369 381
- JP-A- H0 762 472
- JP-A- 10 043 224
- JP-A- 2004 010 997
- JP-A- 2005 237 962
- JP-A- 2005 305 085
- JP-A- 2007 244 852
- JP-B2- 3 300 684
- JP-U- H0 253 716
- JP-U- 59 135 717
- US-A- 2 596 038
- US-A- 3 219 032
- US-A1- 2006 155 233

## Description

### Technical Field

The present invention relates to a device for correcting hallux valgus used for correction when hallux positioned in an innermost portion of toes is deformed or valgus and a producing method thereof.

### Background Art

Hallux valgus is a deformation in which hallux is valgus in a " 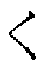 shape" in the first metatarsophalangeal joint and is an ailment that very frequently occurs particularly among women. If the deformation advances, a gait disorder is caused and daily life is extremely obstructed.

In the treatment of hallux valgus, it is important to correct the deformed hallux valgus horn and conservative treatment or surgical treatment is applied. If the deformation is large, surgical treatment is applied, but there is no operation method applicable to any kind of deformation and it is vital to treat hallux valgus before the deformation reaches an advanced stage.

In addition to the treatment by surgical operation, device treatment using a jig for the purpose of correcting hallux valgus is known. The device treatment includes an arch support (insole) used while walking and a correcting device.

For example, Patent Literature 1 discloses a preventive or correcting means for an open foot or the like by the arch support (insole). However, while the means disclosed in Patent Literature 1 is effective for pain, it is difficult for the means to correct deformation.

Patent Literatures 2, 4, and 9, for example, disclose a correcting device with a supporter. Patent Literatures 5, 6 and 8 disclose a correcting instrument made of a tubular body and a plate piece or elastic pad. Patent Literature 7 discloses a correcting instrument using a shape memory alloy that maintains an arch shape with a mesh made of Ti-Ni shape memory alloy wire.

Unfortunately, however, a conventional correcting device does not have a sufficient correcting force and a straightening effect thereof is not sufficient just by being attached as a splint, posing a problem that the correcting device is insufficient for treatment of hallux valgus or preventive treatment.

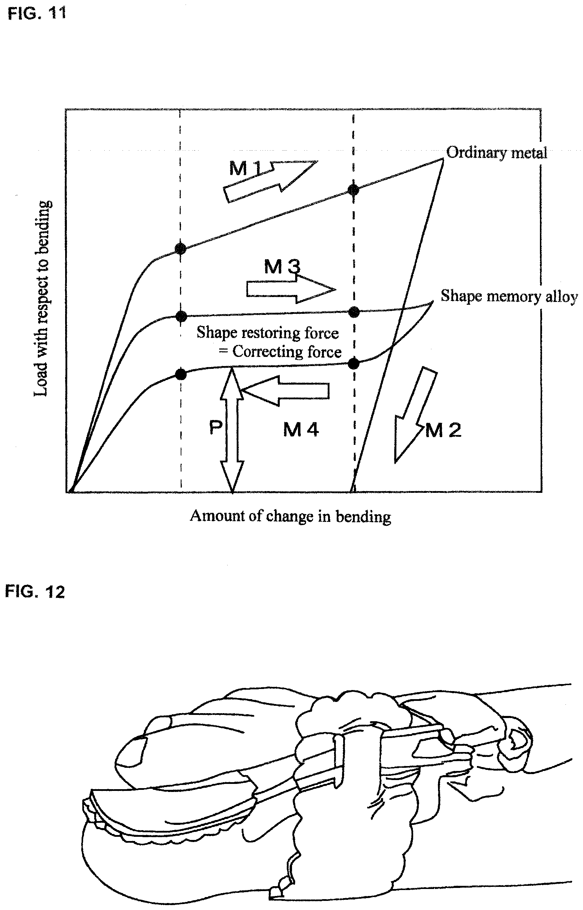

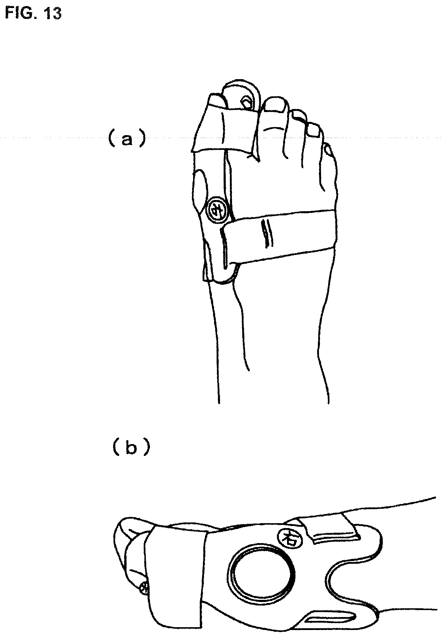

For example, Fig. 12 is a diagram showing an example of a tool for correcting hallux valgus. Fig. 13 is a diagram showing another example of the tool for correcting hallux valgus. The tools for correcting hallux valgus shown in Figs. 12 and 13 are correcting tools intended for night sleeping and is not considered for walking. Particularly, the tool for correcting hallux valgus shown in Fig. 12 can gradually hold hallux in an appropriate position by adjusting the fixed position of a leather belt, but unfortunately standing up/walking is not supported because the tool is made of a thermoplastic plate. In addition, a tool for correcting hallux valgus for daytime walking is known, but unfortunately, a tractive force can be adjusted by adjusting a belt linked from a finger bandage covering hallux and it is necessary for the user to adjust the tractive force.

Further, for example, the Clinical Practice Guideline for Hallux Valgus (Hallux Valgus Guideline Drawing Committee of Clinical Practice Guideline Committee of the Japanese Orthopaedic Association) describes that "though a straightening effect of deformation of a slight degree can be expected when attached, whether the deformation is improved when not attached cannot be concluded" and thus, even today, there is currently no effective device treatment intended for correction.

### Citation List

### Patent Literature

Patent Literature 1: Japanese Patent Application Laid-Open No. 2005-13682
Patent Literature 2: Japanese Patent No. 3748996
Patent Literature 3: Japanese Patent Application Laid-Open No. 2001-29374
Patent Literature 4: Japanese Patent Application Laid-Open No. 2001-299792
Patent Literature 5: Japanese Patent No. 2964405
Patent Literature 6: Japanese Patent Application Laid-Open No. 2008-178436
Patent Literature 7: Japanese Patent Application Laid-Open No. 8-10278
Patent Literature 8: WO2009/144967A1
Patent Literature 9: US2006/0155233A1
Patent Literature 10: JP H07-62472

### Summary of Invention

### Technical Problem

The present invention has been made in view of the above problems and a subject thereof is to provide a device for correcting hallux valgus that can easily be attached and exerts a large correcting force to correct hallux that is deformed and valgus by using a shape memory alloy.

### Solution to Problem

Features of the present invention as a means for solving the above subject include the following:
According to a first aspect of the present invention there is provided a device for correcting hallux valgus, comprising: a correcting plate formed of a shape memory alloy; and an attachment member formed of a cloth-like body to attach the correcting plate; characterised by: a flat fixed plate, configured to be positioned in a nail portion of the hallux; wherein: the correcting plate is formed from a Cu-Al-Mn system shape memory alloy having superelastic effect; the flat fixed plate is formed of a Cu-Al-Mn system shape memory alloy having superelastic effect; the correcting plate (11) has an opening, formed in a portion of the connecting plate which corresponds to a first metatarsophalangeal joint, for correcting the hallux by using a periphery of the opening as a supporting point; and the flat fixed plate is configured to apply a force in a direction opposite to an inner rotation to which the hallux valgus is subject.

Further, in the device for correcting hallux valgus according to the present invention, the correcting plate is further attached to the body portion of the foot and the body portion of the hallux by the attachment member from the hallux inner side direction.

Further, in the device for correcting hallux valgus according to the present invention, the correcting plate is further attached to the body portion of the foot by the first attachment member and the body portion of the hallux by the second attachment member from the hallux inner side direction.

Further, in the device for correcting hallux valgus according to the present invention, the correcting plate further has a pad made of a flexible material placed in the opening.

Further, in the device for correcting hallux valgus according to the present invention, the attachment member is further formed of an elastic body.

Further, in the device for correcting hallux valgus according to the present invention, the device for correcting hallux valgus has the correcting plate and the fixed plate integrated therein.

Further, in the device for correcting hallux valgus according to the present invention, the Cu-Al-Mn system shape memory alloy is formed of an alloy containing Al: 3 to 10% by mass, Mn: 5 to 20% by mass, and copper and inevitable impurities as a rest and to which one or two or more selected from Ni, Co, Fe, Ti, V, Cr, Si, Nb, Mo, W, Sn, Sb, Mg, P, Be, Zr, Zn, B, C, Ag, and misch metal may be added 0.001 to 10% by mass in total.

According to a second aspect of the present invention there is provided a method of producing a device embodying the first aspect of the present invention, the method comprising: using a correcting plate that is formed of a Cu-Al-Mn system shape memory alloy containing Al: 3 to 10% by mass, Mn: 5 to 20% by mass, and copper and inevitable impurities as a rest and to which one or two or more selected from Ni, Co, Fe, Ti, V, Cr, Si, Nb, Mo, W, Sn, Sb, Mg, P, Be, Zr, Zn, B, C, Ag, and misch metal are added 0.001 to 10% by mass in total.

Further, in the method of producing a device for correcting hallux valgus according to the present invention, the Cu-Al-Mn system shape memory alloy is further formed by forming a plate material by cold rolling of a two-phase structure of α + β and, after the plate material being formed into a predetermined shape, by carrying out solution heat treatment in a temperature range of 700 to 950°C in a time range of 0.01 to 1 hour and further, aging treatment in the temperature range of 50 to 250°C in the time range of 0.1 to 1 hour.

### Advantages Effects of Invention

Characteristic effects shown below are achieved by the present invention as a means for solving the above subject.

By virtue of a device for correcting hallux valgus according to the present invention, the device for correcting hallux valgus can easily be attached and correct hallux valgus or hallux varus with a large correcting force by using a shape memory alloy.

Further, by virtue of a method of producing a device for correcting hallux valgus according to the present invention, a device for correcting hallux valgus using a shape memory alloy that allows to be easily attached and exerts a large correcting force can easily be produced.

### Brief Description of Drawings

Fig. 1 is a diagram illustrating the configuration of a device for correcting hallux valgus which does not embody the present invention, but is useful for understanding it.
Fig. 2 is a diagram showing a state of a foot of hallux valgus.
Fig. 3 is a diagram showing the device of Fig. 1. Fig. 3(a) shows a correcting plate, Fig. 3(b) is a perspective view of a foot to which the correcting plate is attached, and Fig. 3(c) is a diagram schematically showing a force of the device for correcting hallux valgus acting on the hallux.
Fig. 4 is a diagram showing a modification of the device of Fig. 1, which does not embody the present invention. Fig. 4(a) shows a correcting plate, Fig. 4(b) is a perspective view of a foot to which the correcting plate is attached, and Fig. 4(c) is a diagram schematically showing a force of the device for correcting hallux valgus acting on the hallux.
Fig. 5 is a diagram showing the state in which the device of Fig. 4 is attached to hallux valgus in a very aggravated state.
Fig. 6 is a diagram showing a modification of the device of Fig. 4, which does not embody the present invention. Fig. 6(a) shows a correcting plate, Fig. 6(b) is a plan view of a foot to which the correcting plate is attached, and Fig. 6(c) is a side view of the foot to which the correcting plate is attached.
Fig. 7 is a diagram illustrating the state of a foot of involuted hallux valgus. Fig. 7(a) is a plan view of the foot and Fig. 7(b) is a front view of the foot.
Fig. 8 is a diagram showing an embodiment of the device for correcting hallux valgus according to the present invention. Fig. 8(a) shows a correcting plate, Fig. 8(b) is a perspective view of a foot to which the correcting plate is attached, and Fig. 8(c) is a diagram schematically showing a force of the device for correcting hallux valgus acting on the hallux.
Fig. 9 is a diagram showing a device for correcting hallux valgus which does not embody the present invention, but is useful for understanding it.
Fig. 10 is a diagram showing a device for correcting hallux valgus which does not embody the present invention, but is useful for understanding it.
Fig. 11 is a diagram illustrating an action of a shape memory alloy used for the correcting plate or a fixed plate of the device for correcting hallux valgus according to the present invention.
Fig. 12 is a diagram showing an example of a tool for correcting hallux valgus.
Fig. 13 is a diagram showing another example of the tool for correcting hallux valgus.

### Description of Examples and Embodiments

The best mode for carrying out the present invention will be described below based on the drawings. A so-called person skilled in the art can easily develop another embodiment by altering/modifying the present invention within the scope of claim, these alterations/modifications are contained within the scope of claim, and the description that follows is an example of the best mode in the present invention and does not limit the scope of claim.

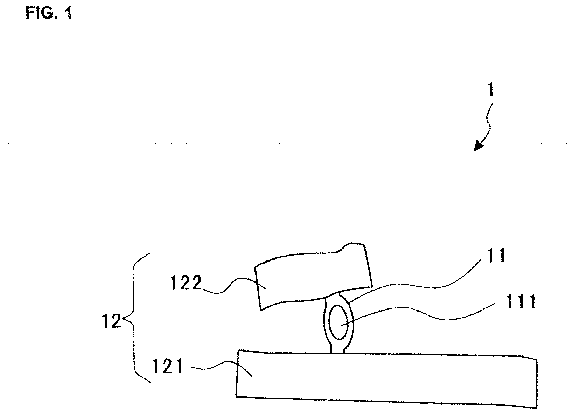

Fig. 1 is a diagram illustrating the configuration of a device for correcting hallux valgus according to a first example which does not embody the present invention.

A device for correcting hallux valgus 1 includes, as shown in Fig. 1, a correcting plate 11 and an attachment member 12.

The correcting plate 11 of the device for correcting hallux valgus 1 is formed of a shape memory alloy. The shape memory alloy is an alloy having one or both of a shape memory effect, which is a property that even if the alloy is deformed at a predetermined temperature (As temperature) or lower, the original shape is restored if the alloy is heated to a predetermined temperature (Af temperature) or higher, and a superelastic effect, which is a property that even if the alloy is deformed at a predetermined temperature (Af temperature) or higher, the original shape is restored if unloaded. If the shape memory effect is used, a correcting force can be applied to hallux valgus 21 by heating. Particularly, deformation/restoration can be achieved by a fixed load or a small load change through the use of superelastic deformation and thus, it is preferable to use the superelastic effect. Therefore, when the hallux valgus 21 is corrected by attaching the correcting plate 11 whose Af temperature is set to the room temperature or lower to a person's foot 2, the hallux valgus 21 is corrected by a force acted on by the original shape being attempted to restore.

Shape memory alloys include a Cu-Al-Mn system, Cu-Zn-Al system, Ti-Mo-Sn system, and Ni-Ti system and an alloy of any system may be used. Patent Literature 10 discloses a Cu-Al-Mn system shape memory alloy.

As shown in Fig. 1, the attachment member 12 of the device for correcting hallux valgus 1 is formed of a cloth-like body for attaching the correcting plate 11. The cloth-like body is thin and can easily be wrapped around the foot 2 or the like. Further, the cloth-like body is preferably elastic. Accordingly, when easily wrapped around the foot 2 or the like, the cloth-like body can be prevented from being shifted or detached.

As shown in Fig. 1, the attachment member 12 uses a separate body type by providing a first attachment member 121 that attaches the correcting plate 11 to a body portion of the foot 2 from an inner side direction of the hallux (big toe) 21 and a second attachment member 122 that attaches the correcting plate 11 to a body portion of the hallux 21. While the attachment member 12 is described as the separate body type separated into two bodies for fixing in front and rear portions, the correcting plate 11 may be attached to the body portion of the foot 2 and the body portion of the hallux 21 by an integrated attachment member from the inner side direction of the hallux 21. The integrated mode includes a supporter type and a socks type. As the device for correcting hallux valgus 1 according to the present invention, any device capable of fixing the body portion of the foot 2 and the portion of the hallux 21 is enough. The attachment member 12 and the correcting plate 11 are integrated by heating or an adhesive. Alternatively, a pocket 123 like a cavity may be provided in the attachment member 12 to insert the correcting plate 11 into the pocket 123 for integration. Accordingly, the correcting plate 11 can be maintained attached to the foot 2 without being shifted after attachment. Moreover, the correcting plate 11 can be attached to the foot 2 in close contact by providing elasticity to the attachment member 12. By adopting the cloth-like body, the attachment member 12 can be made easier to attach. The attachment is achieved by providing and securing a surface fastener. The surface fastener may be secured by hooking on a bandage stop or the cloth-like body may be bound.

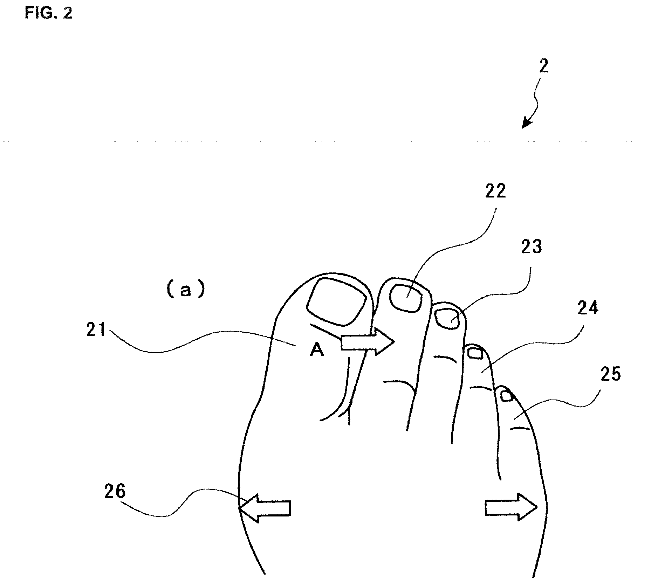

Fig. 2 is a diagram showing a state of a foot of hallux valgus.

If the foot 2 has the hallux valgus 21, as indicated by an arrow A, the hallux 21 is pressed toward the inner side due to pressure of worn shoes or the like and other toes of the foot 2 are likewise subject to a similar pressure. Then, a first metatarsophalangeal joint (joint) 26 of the foot 2 is conversely deformed like being pressed out to the outer side of the foot 2. Therefore, the device for correcting hallux valgus 1 attempts to restore the original state before the deformation.

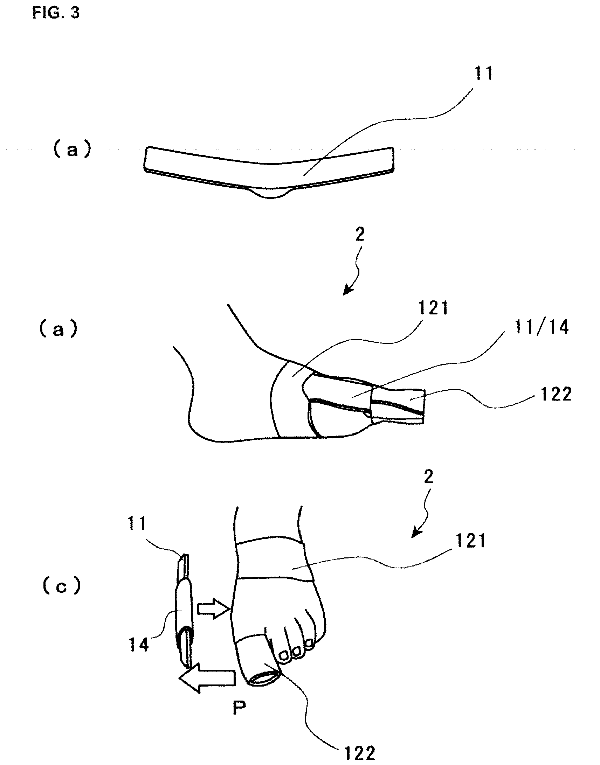

Fig. 3 is a diagram showing the device of Fig. 1. Fig. 3(a) shows a correcting plate, Fig. 3(b) is a perspective view of a foot to which the correcting plate is attached, and Fig. 3(c) is a diagram schematically showing a force of the device for correcting hallux valgus acting
on the hallux.

If the device for correcting hallux valgus 1 using the correcting plate 11 as shown in Fig. 3(a) is attached to the foot 2, the device for correcting hallux valgus 1 is attached as shown in Fig. 3(b). The correcting plate 11 can be attached with stability by adopting a 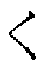 shape along the shape of the foot. In addition, an elastic member such as a sponge 14 may be arranged between the correcting plate 11 and the first metatarsophalangeal joint 26. Further, as shown in Fig. 3(c), the elastic member such as the sponge 14 may be formed in a tabular shape so that the correcting plate 11 is passed through the sponge 14. Accordingly, even if the correcting plate 11 abuts the first metatarsophalangeal joint 26 of the hallux valgus 21, the foot 2 will not be injured or cause pain. If attached to the foot 2 at this point, as indicated by an arrow P in Fig. 3(c), a force acts in a direction pulling the hallux 21 to the outer side of the foot 2 due to the superelastic effect of the device for correcting hallux valgus 1 using a shape memory alloy. Accordingly, deformation of the hallux 21 can be prevented or deformation can be eliminated to restore the original form by pulling the hallux 21 in the direction opposite to the pressure by shoes or the like.

Further, though not illustrated, an elastic member is arranged in the attachment member 12 at the sole so that the height when shoes are worn is adjusted. Accordingly, the promotion of deformation of the foot 2 being corrected by raising the instep of the foot 2 can be prevented.

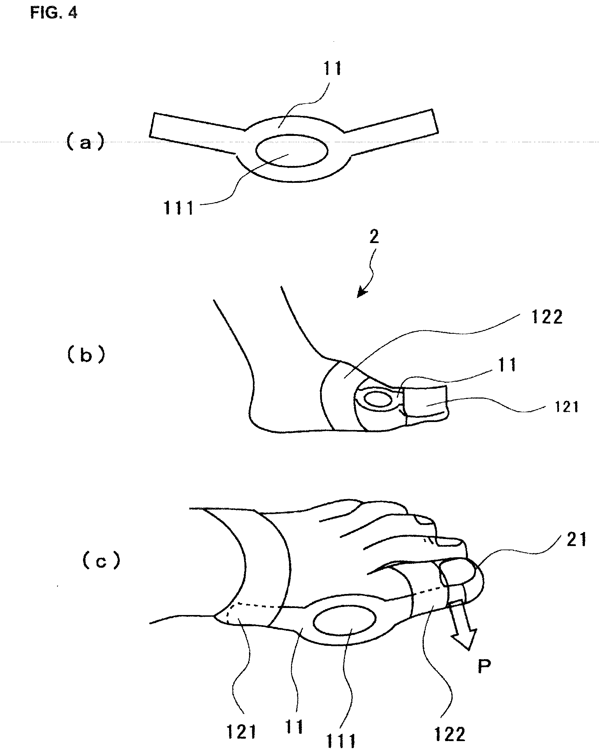

Fig. 4 is a diagram showing a modification of the device of Fig. 1. Fig. 4(a) shows a correcting plate, Fig. 4(b) is a perspective view of a foot to which the correcting plate is attached, and Fig. 4(c) is a diagram schematically showing a force of the device for correcting hallux valgus acting on the hallux.

As shown in Fig. 4(a), the correcting plate 11 has an opening 111 formed in a portion abutting the first metatarsophalangeal joint 26. By abutting the opening 111 on a portion of the hallux valgus 21 projecting to the outer side from the foot 2, the correcting plate 11 can be attached to the foot 2 in close contact by the attachment member 12. Further, a pad made of a foam such as sponge or a flexible material of an elastic body such as rubber is provided in the opening 111. Accordingly, even if the correcting plate 11 abuts the first metatarsophalangeal joint 26 of the hallux valgus 21, the foot 2 will not be injured or cause pain.

As shown in Fig. 4(b), the second attachment member 122 is caused to attach the correcting plate 11 of the device for correcting hallux valgus 1 having the correcting plate 11 to the hallux (big toe) 21 using the periphery of the opening 111 as a supporting point. Thus, as shown in Fig. 4(c), a force acts in the direction of an arrow P to pull the hallux 21 to the inner side in the direction away from the foot 2. Accordingly, the foot 2 of the hallux valgus 21 is corrected, the pressed hallux 21 is prevented from moving in the direction to become the hallux valgus 21, or deformation is eliminated to restore the original form.

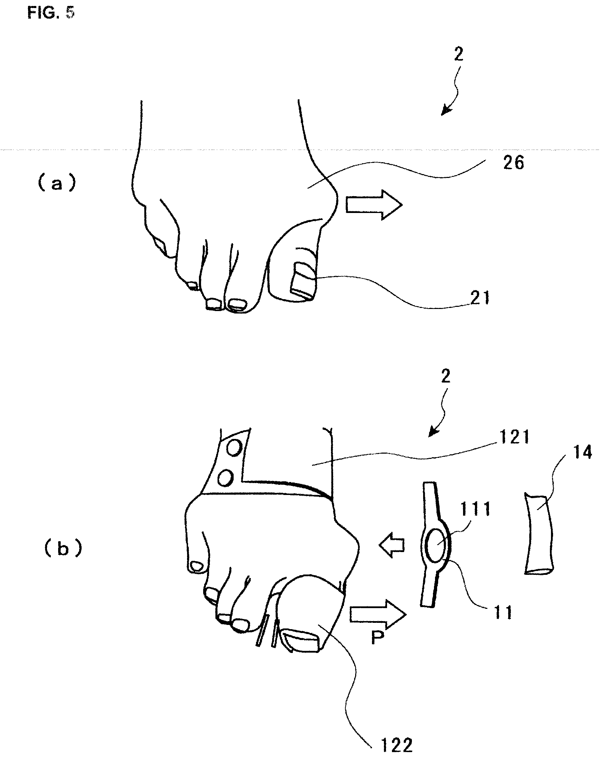

Fig. 5 is a diagram showing the state in which the device of Fig. 4 is attached to hallux valgus in a very aggravated state.

As shown in Fig. 5(a), the first metatarsophalangeal joint 26 projects significantly to the outer side from the foot 2 in the hallux valgus 21 in a very aggravated state. In such a state, it is difficult to attach the correcting plate 11 in a plane shape and even if attached, the correcting plate 11 abuts the first metatarsophalangeal joint 26, which may cause damage or pain or decrease the straightening effect due to displacement of the correcting plate 11. For the above hallux valgus 21, it is preferable to use the device for correcting hallux valgus 1 using the correcting plate 11 having the opening 111. Further, it is preferable to arrange an elastic member such as the sponge 14 between the correcting plate 11 and the first metatarsophalangeal joint 26. Accordingly, as shown in Fig. 5(b), a force acts in the direction of an arrow P to pull the hallux 21 to the inner side in the direction away from the foot 2. Accordingly, the foot 2 of the hallux valgus 21 is corrected, the pressed hallux 21 is prevented from moving in the direction to become the hallux valgus 21, or deformation is eliminated to restore the original form.

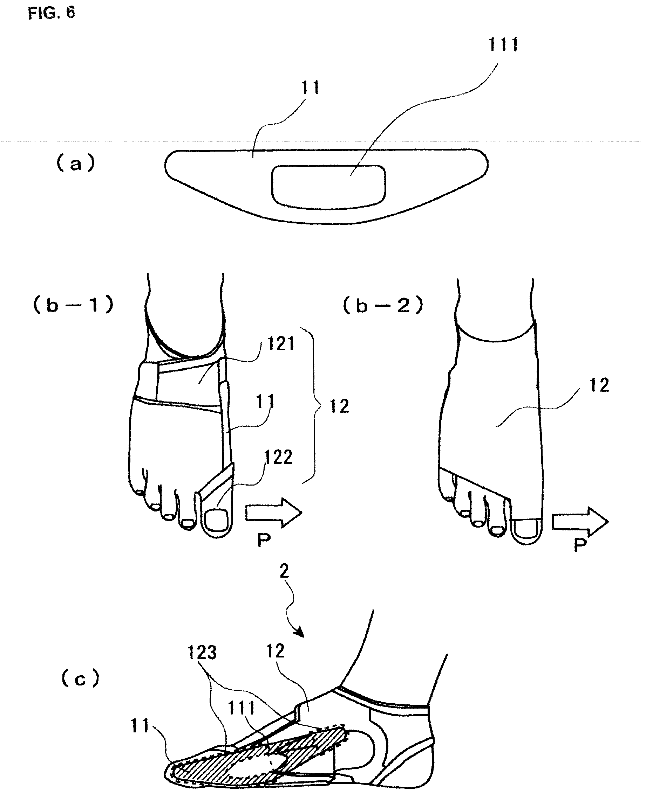

Fig. 6 is a diagram of a modification of the device of Fig. 4. Fig. 6(a) shows a correcting plate, Fig. 6(b-1) is a plan view of a foot to which the device for correcting hallux valgus of separate body type using the correcting plate is attached, Fig. 6(b-2) is a plan view of the foot to which the device for correcting hallux valgus of integrated type using the correcting plate is attached, and Fig. 6(c) is a side view of the foot to which the device for correcting hallux valgus of separate body type using the correcting plate is attached.

As shown in Fig. 6(a), the correcting plate 11 has the opening 111 formed in a portion abutting the first metatarsophalangeal joint 26 and also the opening 111 is linear on one side and elliptic on the opposite side. Accordingly, as shown in Fig. 6(b-1), when the device for correcting hallux valgus 1 of separate body type is used, the correcting plate 11 is attached to a portion close to the instep of the foot 2 by using the attachment member 12 of the second attachment member 122, instead of at the sole where the shape is undulating with the arch of the foot by fitting to the shape of the foot 2.

As shown in Fig. 6(b-2), the attachment member 12 may be of integrated type that attaches the device for correcting hallux valgus 1 to the body portion of the foot 2 and the hallux portion at the same time. Accordingly, the attachment of the device for correcting hallux valgus 1 to the foot 2 is made easier and the displacement of the device for correcting hallux valgus 1 due to motion of the foot 2 can be prevented. The device for correcting hallux valgus 1 of integrated type is a supporter type like an exercise tool and can prevent further application of pressure of deformation to the hallux 21 or eliminate deformation to restore the original form when exercise is done such as walking and running. Accordingly, as shown in Figs. 6(b-1) and 6(b-2), a force acts on the direction of an arrow P to pull the hallux 21 to the inner side in the direction away from the foot 2. Accordingly, the foot 2 of the hallux valgus 21 is corrected, the pressed hallux 21 is prevented from moving in the direction to become the hallux valgus 21, or deformation is eliminated to restore the original form.

As shown in Fig. 6(c), the attachment member 12 is provided with the pocket 123 causing the correcting plate 11 to attach, instead of being integrated with the correcting plate 11. By inserting the correcting plate 11 into the pocket 123, the deformation of the hallux 21 can be prevented by pulling the hallux 21 in the direction opposite to the pressure by shoes or the like or the deformation can be eliminated to restore the original form.

By changing the number of the correcting plates 11 inserted into the pocket 123 or the type thereof, the strength to pull the hallux 21 in the opposite direction can be adjusted.

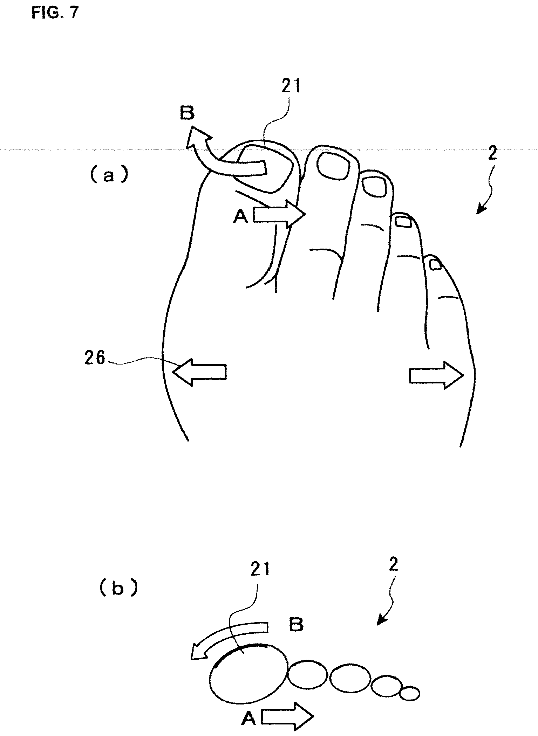

Fig. 7 is a diagram illustrating the state of a foot of involuted hallux valgus. Fig. 7(a) is a plan view of the foot and Fig. 7(b) is a front view of the foot.

If, as described above, the foot 2 has the hallux valgus 21, as indicated by an arrow A, the hallux 21 is pressed toward the inner side by the pressure of worn shoes or the like and other toes of the foot 2 are likewise subject to a similar pressure. Further, as shown in Fig. 7(a), if the foot 2 has the hallux valgus 21, as indicated by an arrow B, the hallux 21 is frequently deformed by being subject to pressure to cause the hallux valgus 21 to involute. The inner rotation occurs, as shown in Fig. 7(b), from the upper portion of the foot 2 toward the lower sole. This also causes a large deformation in the toes and bone of the foot 2 and thus, an inner rotation correction is also needed.

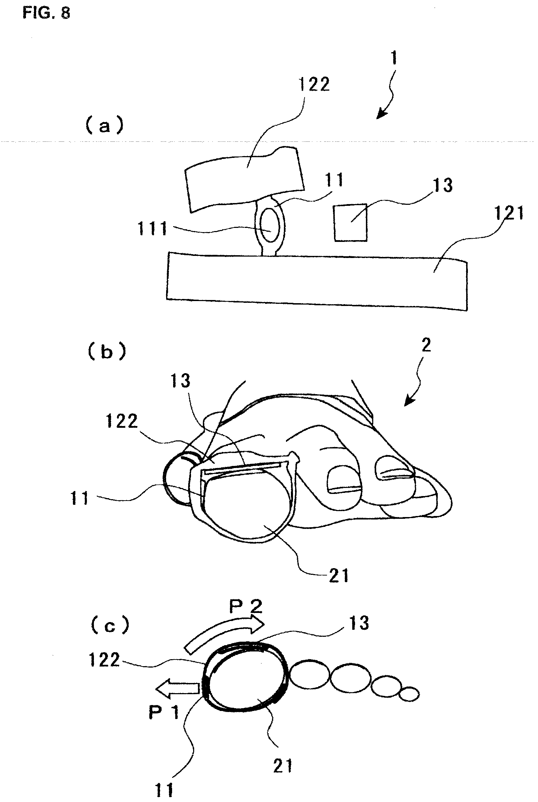

Fig. 8 is a diagram showing an embodiment of a device for correcting hallux valgus according to the present invention. Fig. 8(a) shows a correcting plate, Fig. 8(b) is a perspective view of a foot to which the correcting plate is attached, and Fig. 8(c) is a diagram schematically showing a force of the device for correcting hallux valgus acting on the hallux.

The device for correcting hallux valgus 1 according to the present invention has, as shown in Fig. 8(a), a fixed plate 13, in addition to the correcting plate 11.

The state exerted by the fixed plate 13 is shown as a perspective view of the foot 2 to which the fixed plate 13 is attached in Fig. 8(b) and as a front view of the foot 2 to which the fixed plate 13 is attached in Fig. 8(c).

As shown in Fig. 8(c), a force acts in the direction of an arrow P1 due to the correcting plate 11 and the foot 2 of the hallux valgus 21 can be corrected by pulling the hallux 21 to the inner side in the direction away from the foot 2. Further, the plane fixed plate 13 is attached to a nail portion of the hallux 21 or the whole hallux 21 so that a force acts in the direction of an arrow P2 and an inner rotation correction is made by applying pressure to rotate in the direction opposite to the inner rotation to which the hallux 21 is subject.In an example which does not embody the present invention, the fixed plate 13 may be made of any hard material such as a plastic, resin, and metal. Instead of attaching the fixed plate 13, hard cloth may be fitted to a portion of the attachment member 12 to make the portion hard to use the attachment member 12 in place of the fixed plate 13.

The fixed plate 13 is attached by the attachment member 12 here, but may be integrated with the correcting plate 11 to create a shape memory alloy in a superelastic state. By creating a shape memory alloy in a superelastic state, a rotating force can be exerted on the hallux 21 when pressed even if the shape memory alloy is shifted from the attachment member 12. Further, a superelastic shape restoring force can be changed to a correcting force.

The fixed plate 13 may be a shape memory alloy in a martensite phase state. A shape memory alloy in a martensite phase state is flexible so that an appropriate shape in accordance with the shape of the foot can be provided.

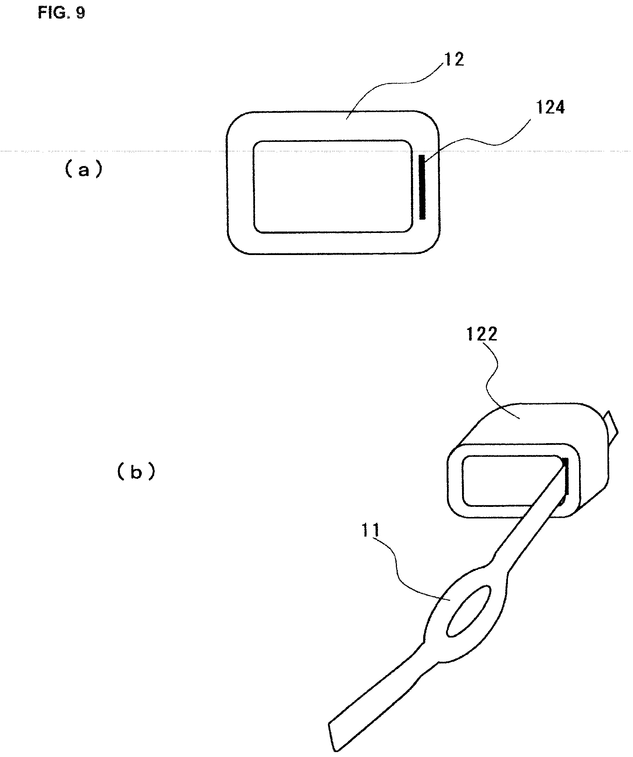

Fig. 9 is a diagram showing a device for correcting hallux valgus according to a second example which does not embody the present invention.

The device for correcting hallux valgus 1 of Fig. 9 uses elastic rubber as the attachment member 12. The device for correcting hallux valgus 1 can be attached to the foot 2 easily and reliably by the rubber. Further, the correcting plate 11 can also be attached easily by providing a slit 124 in a portion of the rubber and inserting an end of the correcting plate 11 through the slit 124.

The rubber may be the integrated attachment member 12, but may be both or one of the first attachment member 121 and the second attachment member 122. Particularly, by using rubber for the second attachment member 122, the second attachment member 122 can easily be attached by inserting the tip of the hallux 21.

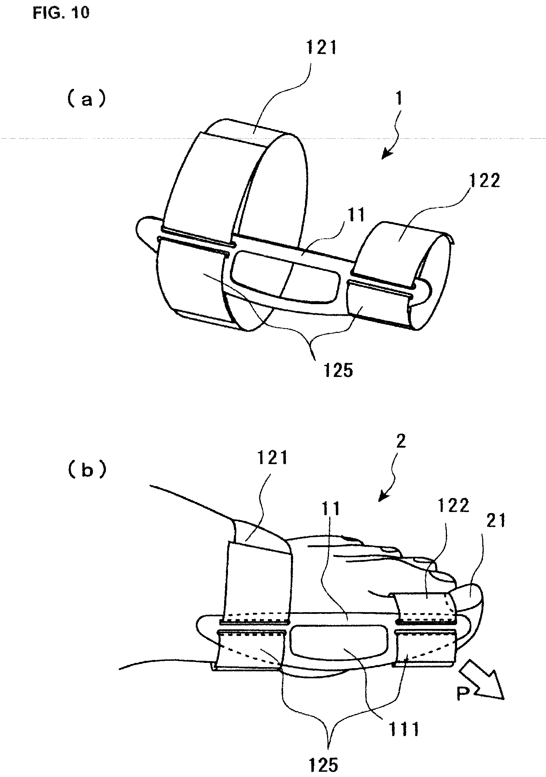

Fig. 10 is a diagram showing a device for correcting hallux valgus according to a third example which does not embody the present invention.

The device for correcting hallux valgus 1 of Fig. 10 uses a surface fastener 125 by adding to elastic bodies such as rubber of the first attachment member 121 and the second attachment member 122 as the attachment member 12. The device for correcting hallux valgus 1 can be attached to the foot 2 easily and reliably by the surface fastener 125.

The surface fastener 125 may be added to the attachment member 12 in which a cloth-like body is integrated or to both or one of the first attachment member 121 and the second attachment member 122 in which the cloth-like body is separated. Particularly, by using the surface fastener 125 for the second attachment member 122, the second attachment member 122 can be attached to a thin portion at the tip of the hallux 21 easily in a short time. Accordingly, a force acts in the direction of an arrow P to pull the hallux 21 to the inner side in the direction away from the foot 2 so that the foot 2 of the hallux valgus 21 can be corrected.

Fig. 11 is a diagram illustrating an action of a shape memory alloy used for the correcting plate 11 or the fixed plate 13 of the device for correcting hallux valgus 1 according to the present invention. The horizontal axis represents the amount of change with respect to bending and the vertical axis represents the load with respect to bending.

An ordinary metal is initially deformed elastically in accordance with the amount of change in bending and then enters a region of plastic deformation and is, as indicated by an arrow M1, deformed plastically. If the application of load is stopped before breakdown, as indicated by an arrow M2, the amount of change is not restored to the original amount of change as the load decreases, leaving the amount of change for plastic deformation.

In the superelastic effect of a shape memory alloy, by contrast, the alloy is initially deformed elastically in accordance with the amount of change in bending and then, as indicated by an arrow M3, the amount of change increases while the load remains almost constant without entering the region of plastic deformation. If the application of load is stopped before breakdown, as indicated by an arrow M4, the amount of change decreases while the load remains constant and the amount of change is restored to the original amount of change, leaving no amount of change for plastic deformation. The superelastic phenomenon acts as a load as a shape restoring force. Further, the load can be used as a correcting force of the hallux valgus 21 and hallux varus 21. Superelastic behavior in Fig. 10 frequently manifests itself in single crystals and a slight rise in load is observed in polycrystal materials with respect to deformation, but the rise in load is very small when compared with an ordinary metal and the correcting force does not depend on the degree of deformation of the hallux valgus 21 very much and thus, an appropriate straightening effect can be achieved. Therefore, deformation of the hallux valgus 21 can be prevented or the deformation can be eliminated to restore the original form by acting a superelastic correcting force for the purpose of correcting the hallux valgus 21 and the hallux varus 21 by using a shape memory alloy for the correcting plate 11 and the fixed plate 13 of the device for correcting hallux valgus 1 according to the present invention to pull and rotate the hallux 21 in the opposite direction.

A Cu-Al-Mn system alloy is used as the shape memory alloy. The Cu-Al-Mn system alloy is obtained at low cost, is highly processible, and can obtain a large correcting force. The Cu-Al-Mn system alloy includes an alloy in which Al is contained 3 to 10% by mass and Mn is contained 5 to 20% by mass and to which copper and a ternary system of impurities and if necessary, one or two or more selected from Ni, Co, Fe, Ti, V, Cr, Si, Nb, Mo, W, Sn, Sb, Mg, P, Be, Zr, Zn, B, C, Ag, and misch metal are added 0.001 to 10% by mass in total.

The Cu-Al-Mn system alloy having the shape memory effect and superelastic effect changes to a β-phase (b.c.c) single phase at high temperature and to a two-phase structure of β + α (f.c.c) at low temperature. 3% or more of Al is needed for the generation of the β single phase, but an excessive quantity of Al exceeding 10% makes the Cu-Al-Mn system alloy brittle. Preferably, the Al content is set to the range of 6 to 10%.

The addition of Mn enables the existence of the β phase on a low Al side and improves cold workability of the Cu-Al-Mn system alloy. Such an effect becomes conspicuous when 5% of Mn or more is contained, but excessive addition of Mn exceeding 20% adversely affects the shape memory effect or the superelastic effect by lowering the martensite transformation temperature. Preferably, the Mn content is set to the range of 8 to 12%.

As other addition ingredients, one or two or more selected from Ni, Co, Fe, Ti, V, Cr, Si, Nb, Mo, W, Sn, Sb, Mg, P, Be, Zr, Zn, B, C, Ag, and misch metal may be added 0.001 to 10% by mass in total.

Ni, Co, Fe, Sn, Sb, and Be produce an action of strengthening a matrix and Ti immobilizes N and O for detoxication. W, V, Nb, Mo, and Zr are components effective for improvement of hardness and abrasion resistance. Cr improves abrasion resistance and corrosion resistance, Si improves corrosion resistance, Mg improves hot workability and ductility, and P and misch metal are added as a deoxidizer and also contribute to ductility improvement. Zn raises the shape memory temperature, B and C strengthen the grain boundary to improve workability and ductility, and Ag contributes to improvement of cold workability. Therefore, a plate material is formed by cold rolling of a two-phase structure of α + β while performing process annealing in the temperature range of 500 to 700°C and after the predetermined shape is formed, solution heat treatment at 700 to 950°C in the time range of 0.01 to 1 hour and further, aging treatment at 50 to 250°C in the time range of 0.1 to 1 hour are carried out.

A shape memory alloy has the shape memory effect that restores the original shape by heating and superelasticity that restores the original shape after unloading of stress and the Cu-Al-Mn system alloy preferably show superelasticity at room temperature.

The stress generally increases in an ordinary metal with an increasing deformation, but the rise in stress due to deformation is small in superelasticity and thus, a deforming force does not depend on the degree of deformation of the hallux valgus 21 much and a straightening force can be applied effortlessly.

If the Cu-Al-Mn system alloy is used as the raw material of the correcting plate 11, changes of the straightening force in accordance with environmental changes are small and thus, a stable straightening force is applied for a long period.

### <Example of the producing method of the Cu-Al-Mn alloy>

An alloy of the composition of Al: 8.1 % by mass, Mn: 11.2% by mass, and Cu for the rest is produced by high-frequency induction fusion and a plate material is produced by casting in a mold, hot rolling at 600°C, and cold rolling while performing intermediate annealing at 600°C. The obtained plate is annealed at 600°C for 10 min to form the plate into a predetermined shape. Further, solution heat treatment at 900°C for five min, cold water, and aging treatment at 200°C for 15 min are carried out before superelastic characteristics manifest themselves.

### Reference Signs List

- 1: Device for correcting hallux valgus
- 11: Correcting plate
- 111: Opening
- 12: Attachment member
- 121: First attachment member
- 122: Second attachment member
- 123: Pocket
- 124: Slit
- 125: Surface fastener
- 13: Fixed plate
- 14: Sponge
- 2: Foot
- 21: Hallux (big toe)
- 22: Second toe
- 23: Third toe
- 24: Fourth toe
- 25: Fifth toe
- 26: First metatarsophalangeal joint (joint)

## Claims

1. A device (1) for correcting hallux valgus, comprising:
a correcting plate (11) formed of a shape memory alloy; and
an attachment member (12; 121, 122) formed of a cloth-like body to attach the correcting plate (11);
**characterised by**:
said device further comprising a flat fixed plate (13), configured to be positioned in a nail portion of the hallux (21);
wherein:
the correcting plate (11) is formed from a Cu-Al-Mn system shape memory alloy having superelastic effect;
the flat fixed plate (13) is formed of a Cu-Al-Mn system shape memory alloy having superelastic effect;
the correcting plate (11) has an opening (111), formed in a portion of the connecting plate (11) which, in use, corresponds to a first metatarsophalangeal joint (26), for correcting the hallux (21) by using a periphery of the opening (111) as a supporting point; and
the flat fixed plate (13) is configured to apply a force in a direction opposite to an inner rotation to which the hallux valgus is subject.

2. The device (1) for correcting hallux valgus according to claim 1, wherein the correcting plate (11) is adapted to be attached to a body portion of a foot (2) and a body portion of the hallux (21) by the attachment member (12) from a hallux inner side direction.

3. The device (1) for correcting hallux valgus according to claim 1, wherein the correcting plate (11) is adapted to be attached to the body portion of the foot (2) by a first attachment member (121) and the body portion of the hallux (21) by a second attachment member (122) from the hallux inner side direction.

4. The device (1) for correcting hallux valgus according to any of claims 1 to 3, wherein
the correcting plate (11) has a pad (14) made of a flexible material placed in the opening (111).

5. The device (1) for correcting hallux valgus according to any of claims 1 to 4, wherein
the attachment member (12; 121, 122) is formed of an elastic body.

6. The device (1) for correcting hallux valgus according to any of claims 1 to 5, wherein
the device (1) for correcting hallux valgus has the correcting plate (11) and the fixed plate (13) integrated therein.

7. The device (1) for correcting hallux valgus according to any of claims 1 to 6, wherein
the Cu-Al-Mn system shape memory alloy is formed of an alloy containing Al: 3 to 10% by mass, Mn: 5 to 20% by mass, and copper and inevitable impurities as a rest and to which one or two or more selected from Ni, Co, Fe, Ti, V, Cr, Si, Nb, Mo, W, Sn, Sb, Mg, P, Be, Zr, Zn, B, C, Ag, and misch metal are added 0.001 to 10% by mass in total.

8. A method of producing a device (1) for correcting hallux valgus as claimed in claim 1, the method comprising:
using a correcting plate (11) that is formed of a Cu-Al-Mn system shape memory alloy containing Al: 3 to 10% by mass, Mn: 5 to 20% by mass, and copper and inevitable impurities as a rest and to which one or two or more selected from Ni, Co, Fe, Ti, V, Cr, Si, Nb, Mo, W, Sn, Sb, Mg, P, Be, Zr, Zn, B, C, Ag, and misch metal are added 0.001 to 10% by mass in total.

9. The method of producing a device (1) for correcting hallux valgus according to claim 8, wherein
the Cu-Al-Mn system shape memory alloy is formed by forming a plate material by cold rolling of a two-phase structure of α + β and, after the plate material being formed into a predetermined shape, by carrying out solution heat treatment in a temperature range of 700 to 950°C in a time range of 0.01 to 1 hour and further, aging treatment in the temperature range of 50 to 250°C in the time range of 0.1 to 1 hour.

## Patentansprüche

1. Vorrichtung (1) zum Korrigieren einer Hallux-valgus-Fehlstellung, umfassend:
eine Korrekturplatte (11), die aus einer Formgedächtnislegierung gebildet ist; und
ein Anbringungselement (12; 121, 122), das aus einem stoffartigen Körper gebildet ist, um an der Korrekturplatte (11) angebracht zu sein;
**dadurch gekennzeichnet, dass** die Vorrichtung ferner Folgendes umfasst:
eine flache feststehende Platte (13), die konfiguriert ist, um in einem Nagelteil der Großzehe (21) positioniert zu sein;
wobei:
die Korrekturplatte (11) aus einer Cu-Al-Mn-System-Formgedächtnislegierung gebildet ist, die einen superelastischen Effekt aufweist;
die flache feststehende Platte (13) aus einer Cu-Al-Mn-System-Formgedächtnislegierung gebildet ist, die einen superelastischen Effekt aufweist;
die Korrekturplatte (11) eine Öffnung (111) aufweist, die in einem Teil der Verbindungsplatte (11) gebildet ist, der im Gebrauch einem ersten Zehengrundgelenk (26) entspricht, um die Großzehe (21) unter Verwendung einer Peripherie der Öffnung (111) als Stützpunkt zu korrigieren; und
die flache feststehende Platte (13) konfiguriert ist, um eine Kraft in einer Richtung auszuüben, die einer Innendrehung entgegengesetzt ist, der die Hallux-valgus-Fehlstellung unterliegt.

2. Vorrichtung (1) zum Korrigieren einer Hallux-valgus-Fehlstellung nach Anspruch 1, wobei die Korrekturplatte (11) geeignet ist, um an einem Körperteil eines Fußes (2) und einem Körperteil der Großzehe (21) durch das Anbringungselement (12) von einer innenseitigen Richtung der Großzehe aus angebracht zu sein.

3. Vorrichtung (1) zum Korrigieren einer Hallux-valgus-Fehlstellung nach Anspruch 1, wobei die Korrekturplatte (11) geeignet ist, um an dem Körperteil des Fußes (2) durch ein erstes Anbringungselement (121) und an dem Körperteil der Großzehe (21) durch ein zweites Anbringungselement (122) von einer innenseitigen Richtung der Großzehe aus angebracht zu sein.

4. Vorrichtung (1) zum Korrigieren einer Hallux-valgus-Fehlstellung nach einem der Ansprüche 1 bis 3, wobei die Korrekturplatte (11) ein Polster (14) aufweist, das aus einem biegsamen Material besteht, das in die Öffnung (111) gelegt ist.

5. Vorrichtung (1) zum Korrigieren einer Hallux-valgus-Fehlstellung nach einem der Ansprüche 1 bis 4, wobei das Anbringungselement (12; 121, 122) aus einem elastischen Körper gebildet ist.

6. Vorrichtung (1) zum Korrigieren einer Hallux-valgus-Fehlstellung nach einem der Ansprüche 1 bis 5, wobei die Vorrichtung (1) zum Korrigieren einer Hallux-valgus-Fehlstellung die Korrekturplatte (11) und die feststehende Platte (13) darin integriert aufweist.

7. Vorrichtung (1) zum Korrigieren einer Hallux-valgus-Fehlstellung nach einem der Ansprüche 1 bis 6, wobei die Cu-Al-Mn-System-Formgedächtnislegierung aus einer Legierung gebildet ist, die Al: 3 bis 10 Massenprozent, Mn: 5 bis 20 Massenprozent und Kupfer und unvermeidliche Fremdstoffe als Rest enthält, und zu der ein oder zwei oder mehrere Elemente, das bzw. die aus Ni, Co, Fe, Ti, V, Cr, Si, Nb, Mo, W, Sn, Sb, Mg, P, Be, Zr, Zn, B, C, Ag und Mischmetall ausgewählt wird bzw. werden, insgesamt mit 0,001 bis 10 Massenprozent hinzugefügt wird bzw. werden.

8. Verfahren zum Herstellen einer Vorrichtung (1) zum Korrigieren einer Hallux-valgus-Fehlstellung nach Anspruch 1, wobei das Verfahren den folgenden Schritt umfasst:
Verwenden einer Korrekturplatte (11), die aus einer Cu-Al-Mn-System-Formgedächtnislegierung gebildet ist, die Al: 3 bis 10 Massenprozent, Mn: 5 bis 20 Massenprozent und Kupfer und unvermeidliche Fremdstoffe als Rest enthält, und zu der ein oder zwei oder mehrere Elemente, das bzw. die aus Ni, Co, Fe, Ti, V, Cr, Si, Nb, Mo, W, Sn, Sb, Mg, P, Be, Zr, Zn, B, C, Ag und
Mischmetall ausgewählt wird bzw. werden, insgesamt mit 0,001 bis 10 Massenprozent hinzugefügt wird bzw. werden.

9. Verfahren zum Herstellen einer Vorrichtung (1) zum Korrigieren einer Hallux-valgus-Fehlstellung nach Anspruch 8, wobei die Cu-Al-Mn-System-Formgedächtnislegierung gebildet wird, indem ein Plattenmaterial durch Kaltwalzen einer Zweiphasenstruktur aus α + β, und nachdem das Plattenmaterial in eine vorbestimmte Form gebildet wurde, indem ein Lösungsglühen in einem Temperaturbereich von 700 bis 950 °C in einem Zeitbereich von 0,01 bis 1 Stunde ausgeführt wird, und ferner eine Alterungsbehandlung in dem Temperaturbereich von 50 bis 250 °C in dem Zeitbereich von 0,1 bis 1 Stunde ausgeführt wird, gebildet wird.

## Revendications

1. Dispositif (1) de correction d'hallux valgus, comprenant :
une plaque de correction (11) formée d'un alliage à mémoire de forme ; et
un élément de fixation (12 ; 121, 122) formé d'un corps analogue à du tissu pour fixer la plaque de correction (11) ;
**caractérisé par** :
ledit dispositif comprenant en outre
une plaque plate fixe (13), configurée pour être positionnée dans une partie pointue de l'hallux (21) ;
dans lequel :
la plaque de correction (11) est formée à partir d'un alliage à mémoire de forme de système Cu-Al-Mn ayant un effet superélastique ;
la plaque plate fixe (13) est formée d'un alliage à mémoire de forme de système Cu-Al-Mn ayant un effet superélastique ;
la plaque de correction (11) présente une ouverture (111), formée dans une partie de la plaque de correction (11) qui correspond à une première articulation métatarsophalangienne (26), pour corriger l'hallux (21) en utilisant une périphérie de l'ouverture (111) comme point de support ; et
la plaque plate fixe (13) est configurée pour appliquer une force dans une direction opposée à une rotation intérieure à laquelle l'hallux valgus est soumis.

2. Dispositif (1) de correction d'hallux valgus selon la revendication 1, dans lequel
la plaque de correction (11) est adaptée pour être fixée à une partie de corps d'un pied (2) et une partie de corps de l'hallux (21) par l'élément de fixation (12) à partir d'une direction latérale interne de l'hallux.

3. Dispositif (1) de correction d'hallux valgus selon la revendication 1, dans lequel
la plaque de correction (11) est adaptée pour être fixée à la partie de corps du pied (2) par un premier élément de fixation (121) et à la partie de corps de l'hallux (21) par un second élément de fixation (122) à partir de la direction latérale intérieure de l'hallux.

4. Dispositif (1) de correction d'hallux valgus selon l'une quelconque des revendications 1 à 3, dans lequel
la plaque de correction (11) comporte un coussinet (14) fabriqué en matériau souple placé dans l'ouverture (111).

5. Dispositif (1) de correction d'hallux valgus selon l'une quelconque des revendications 1 à 4, dans lequel
l'élément de fixation (12 ; 121, 122) est formé d'un corps élastique.

6. Dispositif (1) de correction d'hallux valgus selon l'une quelconque des revendications 1 à 5, dans lequel
le dispositif (1) de correction d'hallux valgus comporte la plaque de correction (11) et la plaque fixe (12) intégrées dans celui-ci.

7. Dispositif (1) de correction d'hallux valgus selon l'une quelconque des revendications 1 à 6, dans lequel
l'alliage à mémoire de forme de système Cu-Al-Mn est formé d'un alliage contenant de l'Al pour 3 à 10 % en masse, du Mn pour 5 à 20 % en masse, et du cuivre et des impuretés inévitables en tant que reste, et auquel sont ajoutés un, deux ou plus de deux choisis parmi Ni, Co, Fe, Ti, V, Cr, Si, Nb, Mo, W, Sn, Sb, Mg, P, Be, Zr, Zn, B, C, Ag et du métal de Misch pour 0,001 à 10 % en masse au total.

8. Procédé de fabrication d'un dispositif (1) de correction d'hallux valgus tel que revendiqué dans la revendication 1, le procédé comprenant :
l'utilisation d'une plaque de correction (11) qui est formée d'un alliage à mémoire de forme de système Cu-Al-Mn contenant de l'Al pour 3 à 10 % en masse, du Mn pour 5 à 20 % en masse, et du cuivre et des impuretés inévitables en tant que reste, et auquel sont ajoutés un, deux ou plus de deux choisis parmi Ni, Co, Fe, Ti, V, Cr, Si, Nb, Mo, W, Sn, Sb, Mg, P, Be, Zr, Zn, B, C, Ag et du métal de Misch pour 0,001 à 10 % en masse au total.

9. Procédé de fabrication d'un dispositif (1) de correction d'hallux valgus selon la revendication 8, dans laquelle
l'alliage à mémoire de forme de système Cu-Al-Mn est formé en formant un matériau en plaque par laminage à froid d'une structure biphasique de α + β et, après la formation du matériau en plaque en une forme prédéterminée, en effectuant un traitement thermique en solution dans une plage de températures de 700 à 950°C dans une plage de temps de 0,01 à 1 heure et, de plus, un traitement de vieillissement dans la plage de températures de 50 à 250°C dans la plage de temps de 0,1 à 1 heure.
